# EUROPEAN PATENT SPECIFICATION

(11) **EP 0 513 128 B1**
(45) Date of publication and mention of the grant of the patent: **19.07.1995**
(21) Application number: 91903551.9
(22) Date of filing: 18.01.1991
(51) Int. Cl.: A61M 5/24, A61M 5/19

(54) **APPARATUS FOR MIXING AND INJECTING A MEDICINE**
VORRICHTUNG ZUM MISCHEN UND SPRITZEN EINES MEDIKAMENTS
APPAREIL DE MALANGE ET D'INJECTION D'UN MEDICAMENT

(30) Priority: 22.01.1990 DK 178/90
(43) Date of publication of application: 19.11.1992
(73) Proprietor: NOVO NORDISK A/S, 2880 Bagsvaerd (DK)
(72) Inventor: BONNICHSEN, Frits, DK-3540 Lynge (DK)
(74) Representative: Hansen, Einar Tronier (DK)
(86) International application number: DK9100012
(87) International publication number: WO9110460

(56) References cited:
- EP-A- 0 298 067
- EP-A- 0 327 910
- WO-A-88/07874
- US-A- 1 604 224
- US-A- 2 607 344
- US-A- 2 646 798
- US-A- 4 613 326

## Description

The invention concerns a syringe for mixing a liquid and a medicament in a two-chamber cartridge comprising a barrel having its front end closed by a membrane, its rear end closed by a first piston, and its inner space divided into two compartments by a second piston, and for subsequent dosed injection of the mixture provided, this apparatus comprising,
a tubular housing having an open front end and a rear end,
a piston rod having a front end for engagement with the first piston of the cartridge, and a rear end having a thread,
a dose setting member having a thread engaging the thread of the piston rod so that the total length of the piston rod with the dose setting member may be adjusted by rotating the dose setting member relative to the piston rod,
a locking member which may be screwed into the open front end of the housing leaving space for a needle mounted on this locking member.

The cartridge has a passage between the two chambers, which passage may be opened by a joint displacement of the first and the second piston and the liquid between them in a direction towards the front end of the cartridge, whereafter a further displacement of the second piston presses the liquid through the passage for mixing with the medicament.

Processes and apparatus of this kind are particularly used for ambulatory treatment as the patient himself can mix the medicine before it is injected.

Particularly by easily decomposable medicine substances, these may advantageously be packaged in a two-compartment cartridge, whereby the shelf life will be extended considerably.

An example of such a medicine may be a freeze-dried growth hormone which cannot stand vigorous shaking when mixed, and consequently the mixing should take place shortly before the injection and as carefully as ever possible.

From US 2 646 798 is known a syringe for receiving a two-compartment cartridge for mixing the medicine component stored in the compartments by a continuous linear movement of the piston rod, and by which syringe the mixed medicine may be injected in immediate continuation of the mixing.

From EP-A 0 327 910 is known a syringe by which a dosed injection of medicine may be obtained by successively increasing the length of a piston rod and pressing this piston rod home in a cartridge so that for every increment of the piston rod length a dose corresponding to this increment is injected.

From the specification of EP-A 0 298 069 a device for mixing the content of the compartment of a two-compartment cartridge an for subsequent dosed injection of the mixture is known.

The mixing is performed by the rear piston being moved forwardly in the rear chamber by a screw mechanism pressing the liquid-containing phase into the front chamber with the sensitive phase under a relatively high pressure, and thus the mixing is carried out under pressure in the closed front chamber.

The spiral movement is performed by rotating a button at the end of the device by the fingers, which means that the spiral movement will take place jerkily. This will inevitably disturb the mixing, both as regards shakings and as regards the admission of liquid. Furthermore, the mixing takes place under pressure as the cartridge is sealed by a membrane, which produces unwanted mixing whirls in the mixture.

It is the object of the invention to provide a syringe of the kind described in the opening of this application by which syringe a mixing of the components in the compartments of a two-compartment cartridge may be performed by a continuous movement of the piston and by which the mixing process and the injection process are distinctly independent processes.

This is obtained by a syringe of the kind described in the opening of this application which syringe is characterized in, that the piston rod with the dose setting member is axially displaceable mounted in the rear end of the housing so that the rear end of the piston rod with the dose setting member is pressed out through the rear end of the housing when the cartridge is inserted through the open front end of this housing with its first piston abutting the front end of the piston rod to press this rod backwards, the minimum length of the piston rod with the dose setting member being so that a mixing is just performed in the fully inserted cartridge clamped in the housing by screwing the locking member into the open end of this housing when the piston rod with the dose setting member is pressed home into the housing until the dose setting member abuts a stop on the housing.

By the syringe according to the invention a particularly careful mixing may be obtained, primarily as the forward movement of the piston rod will be a continuous motion, i.e. without breaks when the piston rod is moved forward, e.g. when pushed against a tabletop or the like. Thus, this mixing may be performed without shakings and unwanted variations in the admission of liquid being. This provides a hitherto unknown and good shelf life of the mixture and thus the optimal treatment.

As mentioned in claim 2, by providing the piston rod in a mixing and injecting apparatus with a withdrawal detent the risk of unintended inlet of air by withdrawal, e.g. when setting the dose, is averted.

As mentioned in claim 3, by providing the apparatus with a setting mechanism for determining the forward movement of the piston rod during injection, an accurate dosing and consequently an optimal medical treatment is obtained.

As mentioned in claim 4, by providing the setting mechanism with a scale which is set to zero after each injection the risk of incorrect dosing will be reduced.

As mentioned in claim 5, by preventing the piston rod from rotating when setting the dose, injection of the exact amount is insured.

Finally, as referred to in claim 6, it is appropriate to design the withdrawal detent and the coupling as holders having arms with teeth engaging backwards facing teeth on the piston rod whereby the piston rod on one side is prevented from being passed backwards when the arms are pressed against the piston rod, and on the other side is prevented from being rotated when the holders engage longitudinal tracks in the piston rod.

The invention will now be described in further details with reference to the drawing in which
- Fig. 1: shows a sectional view of an injecting apparatus for manual needle insertion, and where the mixing is completed and the apparatus is ready for setting and injection, and
- Fig. 2: shows a sectional view of an injecting apparatus for automatic needle insertion, where the mixing is completed too, and where the apparatus is ready for setting and injection.

On the drawing two embodiments of the apparatus according to the invention are shown as examples of preferred embodiments.

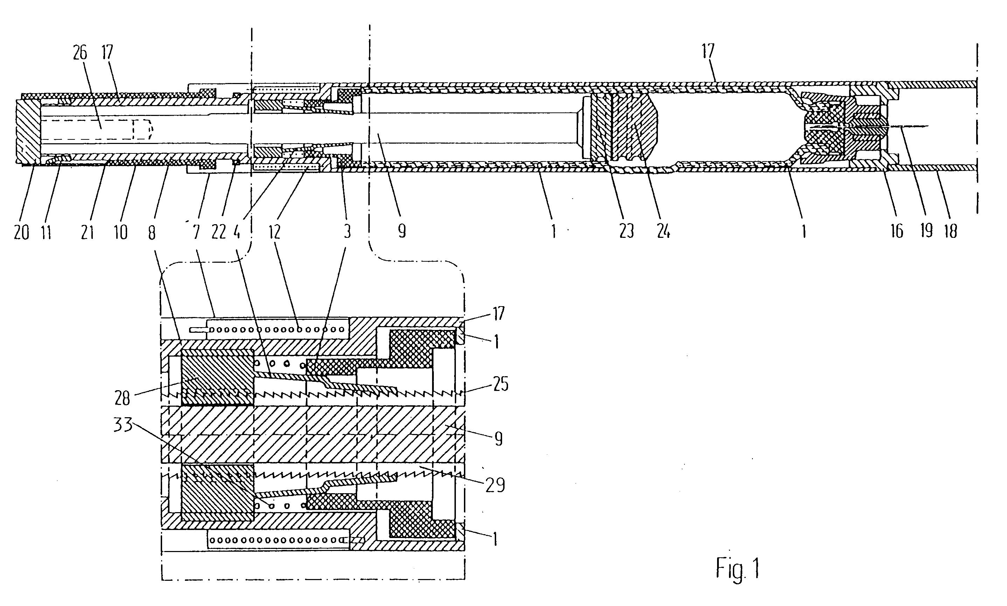

Referring to Fig. 1 a so-called manual mixing and injecting pen will be described in further details below.

The two-compartment cartridge 1 when in its standard storing condition is divided into two compartments, the liquid phase being in the rear chamber, and the medicine phase in the front chamber.

The piston 24 is in a position behind the passage between the two chambers, and the rear piston 23 is at the rear of the cartridge 1. At its front the cartridge is provided with a membrane and a holder for the needle 19, which needle is, however, usually not mounted until after the cartridge has been inserted in the house 17 of the pen.

The house 17 has the form of a tubular case, wherein the cartridge 1 can be inserted from the right in the drawing. When the cartridge 1 has been inserted it is secured by screwing into the house from the front thereof a locking member 16 with the needle 19 penetrating the membrane.

Behind the cartridge 1 a thrust collar 3 has been situated, which thrust collar is provided with a smaller thrust collar which is passed backwards into the house when the cartridge 1 is inserted.

From the enlarged detail drawing it furthermore appears that at its back the house 17 is designed with a case member 8, wherein a locking device 28 having elastic detent arms 4 is mounted. These arms 4 normally extend in axial direction and are at their under side at their ends provided with a cogging sloping forwards.

When during insertion the cartridge 1 is pressed against the thrust collar 3, the arms 4 are pressed downwards when the cartridge is pressed home, and the teeth pass into a barrier mesh with corresponding teeth 25 provided on the outer side of a piston rod 9.

This mesh functions as a one-way detent as the piston rod 9 will then be forwards slidable, but never backwards, thus making suction of air impossible.

The piston 9 is furthermore prevented from rotating about its longitudinal axis, as the arms 4 or the locking device 28 functions as a pawl in a longitudinal track 29 in the piston rod 9, so that the piston rod is made unrotatable, which is necessary in order to accomplish a setting of the dose by a rotational movement.

As appears from the drawing, the piston rod 9 projects over the house 17 and is at its end provided with a thread which can be placed inside, as shown, or outside.

Into this inside thread is screwed a threaded member 26, being at its end fixed to a bottom 20 which extends outside in a cap 10, which again runs outside on the case member 8 of the house 17.

The rear of the case member 8 is provided with a click arm 11 which works with inside longitudinal grooves in the cap 10, and the axial movement of the cap is delimited by these grooves, the grooves providing a stop 21 delimiting the longitudinal displacement of the cap 10.

Finally, a scale 7 actuated by a circumferential spring 12 is mounted, so that when setting the dose by rotating the cap 10, 20, the scale 7 may be detained against a non-shown stop. The scale is released when pulling back the cap, whereby the spring turns the scale to its initial position.

The dosing is set by rotating the cap and is marked by the number of clicks from the click arm 11, thus unscrewing the cap 20 from the piston rod 9. At the same time the scale is rotated in such a way that the dose may be read as compared to a zero on the house 17. Then the injection is carried out by pressing forward the cap 20 until the injection is finished, i.e. when the cap 20 abuts on the case 8. Hereafter the cap 10 is passed back zeroing the scale 7, and a new dose may be set and injected.

The scale further has a stop 22 on the case 8 making it possible to limit the movement and thereby the set dose, the movement of the case 8 in the longitudinal direction being limited.

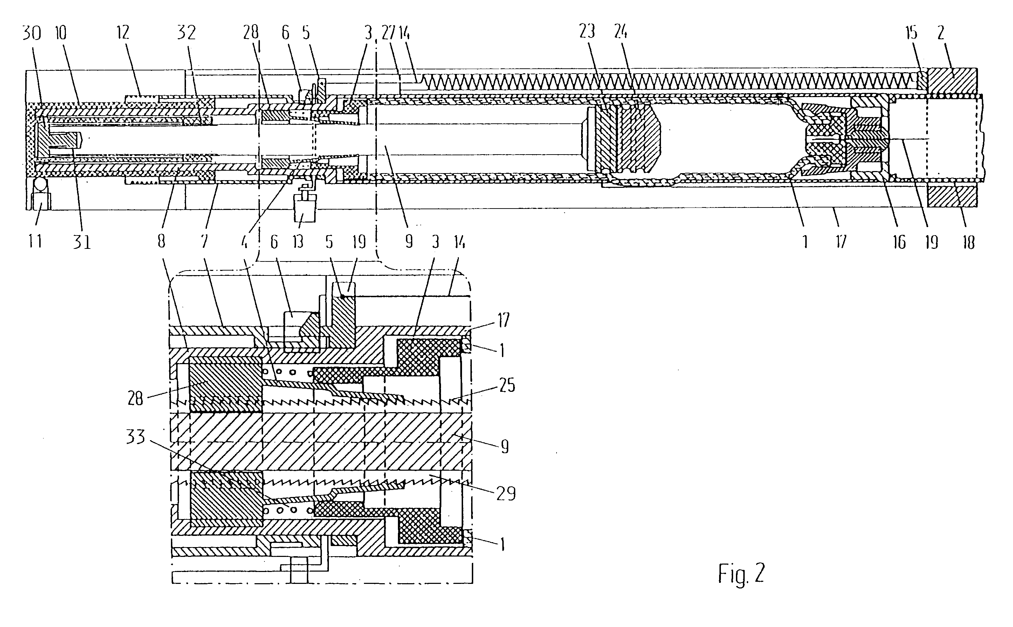

On Fig. 2 is shown an example of an embodiment where the apparatus is also provided with a spring-driven needle insertion device.

In the house 17 there is also a chamber for the cartridge 1, so that it can be inserted from the end and thus influence the thrust collar 3 which again will actuate the detent arms 4 to be thrown into mesh with the teeth 25 of the piston rod 9. The cap 10 is also rotatable in relation to the piston rod 9 via a threaded member 32, engaging an outside thread on the piston rod 9.

Furthermore, a threaded member 31 with a disc 30 is screwed into the end of the piston rod 9 making it possible to preset the maximum forward movement of the piston rod, thus ensuring that the cartridge contains sufficient liquid for the last set dose, as the disc 30 will abut the threaded member 32 when setting a dose bigger than the one left in the cartridge.

In this embodiment the cap is externally supplied with grooves in which a ball 11 can be pressed and function as a click lock. The scale 7 is also provided with a circumferential spring 12 keeping the scale in abutment with a non-shown stop, and which spring is released and returns the scale to zero when after injection the cap 10 is disengaged.

For insertion of the needle 19 all the inner parts of the apparatus are slidable in the house 17. A tension spring 14 is fastened 15 at the front of the house, and at the rear it is fastened to a hook 5 surrounding the case 8, as appears more clearly from the enlarged detail drawing.

In order to release the tightened spring 14 a locking member 6 is mounted, which via a release mechanism 13 can release the hook 5, whereby the needle 19 may be moved forward to be inserted in the skin. The dose preset by the cap 10 is then injectable.

The process for mixing and injecting will be described in further details below.

The cartridge 1 is inserted in the house 17, whereby the piston rod 9 will be passed backwards as the arms 4 are disconnected by means of a compression spring 33. When the cartridge is at the bottom, the thrust collar 3 is acted on and the arms are pressed against the piston rod, as shown in the drawing.

A needle holder 16 is then screwed into the front part of the cartridge to make the air in the system escape through the needle 19 during the mixing. The needle is now mounted and secured.

The mixing is performed by screwing the cap 10 down against the end of the piston rod as shown in the drawing, and by holding the pen in a vertical position and placing the cap in abutment against e.g. a table top the cap may be pressed gently inwardly until it is flush with the house. The mixing is now finished, the pistons 23, 24 being situated abreast of the passage as shown in the drawing.

Referring to Fig. 2, the injection will now be explained.

A cap 18 protecting the needle 19 is pressed inwards. Hereby the spring 14 is compressed and the hook 5 engages the locking member 6. At the same time the cap 10 engages the scale 7 and a presetting may be performed by rotating the cap relative to the piston rod 9. During this rotation the ball lock 11 clicks into the grooves on the cap 10, so that the spring 12 cannot rotate back the cap and the scale. The number of clicks indicates the set dose.

The cap is screwed outwards by the setting and is separated from the piston rod 9 which is clutched by the coupling 3, 4 and 28, 29.

Hereafter the cap 18 may be removed and a space member 2 may be placed against the skin whereupon the release button 13 may be actuated.

The hook 5 will be released and the spring 14 will draw the unit and the hook with it down to a stop 27. The needle will now be injected and the cap 10 will be out of engagement with the scale 7 which is set to zero by the spring 12.

The injection is performed by pressing the cap 10 against the case 8. Hereafter the apparatus and with it the needle 19 may be withdrawn from the skin and the cap may be put on again, whereupon a compressing of the spring, a setting, and an injection may be performed anew.

When the cartridge 1 is empty a normal presetting is performed by rotating the cap 10, and the locking member 16 is removed by being screwed out. Hereafter the cap 10 is pressed forwards whereby a stop (not shown) on the piston rod 9 and/or the compression spring 33 will affect the ring 3 forwards whereby the arms 4 are coupled free and the cartridge may be drawn out. Hereafter a new cartridge may be mounted and the mixing may be performed again as described.

The method performed by the apparatus of Fig. 1 is in principle identic to the one shown in Fig. 2, but the cap is divided into two, the button 20 being screwed against the piston rod 9 before the mixing.
By the method and the apparatus according to the invention the most careful mixing is obtained and the dosing is extremely exact, the optimal medical effect being ensured. Especially by sensitive and expensive hormone preparations this is of decisive importance to the result of the treatment.

## Claims

1. A syringe for mixing a liquid and a medicament in a two-chamber cartridge (1) comprising a barrel having its front end closed by a membrane, its rear end closed by a first piston (23), and its inner space divided into two compartments by a second piston (24), and for subsequent dosed injection of the mixture provided, this apparatus comprising,
a tubular housing (17,8) having an open front end and a rear end,
a piston rod (9) having a front end for engagement with the first piston (23) of the cartridge (1), and a rear end having a thread,
a dose setting member (10,20) having a thread engaging the thread of the piston rod (9) so that the total length of the piston rod (9) with the dose setting member (10,20) may be adjusted by rotating the dose setting member (10,20) relative to the piston rod (9),
a locking member (16) which may be screwed into the open front end of the housing (17) leaving space for a needle (19) mounted on this locking member (16),
**characterized** in that the piston rod (9) with the dose setting member (10,20) is axially displaceable mounted in the rear end of the housing (17,8) so that the rear end of the piston rod (9) with the dose setting member (10,20) is pressed out through the rear end of the housing when the cartridge (1) is inserted through the open front end of this housing (17) with its first piston (23) abutting the front end of the piston rod (9) to press this rod backwards, the minimum length of the piston rod (9) with the dose setting member (10,20) being so that a mixing is just performed in the fully inserted cartridge (1) clamped in the housing (17) by screwing the locking member (16) into the open end of this housing (17) when the piston rod (9) with the dose setting member (10,20) is pressed home into the housing until the dose setting member (10,20) abuts a stop on the housing (17).

2. A syringe according claim 1, **characterized** in that a withdrawal detent (4,25) is provided and is activated when a cartridge is inserted in the housing (17).

3. An apparatus according to claims 1 or 2, **characterized** in, that by injection after the mixing the forward movement of the piston rod (9) is preset by the dose setting member (10,20) being unscrewed from the rear part of the piston rod (9), so that the distance between the dose setting member (10,20) and the stop on the housing (17) is set by rotation of said dose setting member (10,20) relative to the piston rod (9).

4. A syringe according to claim 3, **characterized** in that a scale (7) is by a spring (12) biased towards a zero position in relation to the housing (17) and is coupled to the dose setting member (10,20) to follow the rotation of this member when it is rotated to screw it away from its abutment with the stop, the coupling being released when the dose setting member is pressed home to abutment with the stop on the housing (17).

5. A syringe according to claim 3, **characterized** in that the piston rod (9) is clutched by a coupling (28,29) during the rotation of the dose setting member (10,20).

6. Apparatus according to claim 2, 3 and 5, **characterized** in, that the withdrawal detent (4,25) and the coupling (28,29) are provided as holders (28) having arms (4) with teeth engaging backwards facing teeth (25) on the piston rod (9) whereby the piston rod on one side is prevented from being passed backwards when the arms (4) are pressed against the piston rod (9), and on the other side is prevented from being rotated when the holders (28) engage longitudinal tracks (29) in the piston rod (9).

## Patentansprüche

1. Spritze zum Mischen einer Flüssigkeit und eines Medikaments in einer Zweikammer-Patrone (1) mit einer Trommel, deren vorderes Ende durch eine Membran geschlossen ist, deren hinteres Ende durch einen ersten Kolben (23) geschlossen ist, und deren Innenraum in zwei Fächer durch einen zweiten Kolben (24) geteilt ist, und zur nachfolgenden dosierten Injektion der bereitgestellten Mischung, wobei diese Vorrichtung umfaßt
ein rohrförmiges Gehäuse (17, 8) mit einem offenen vorderen Ende und einem hinteren Ende,
einen Kolbenstab (9) mit einem vorderen Ende zum Eingriff mit dem ersten Kolben (23) der Patrone (1), und einem hinteren Ende mit einem Gewinde,
ein Dosiseinstellelement (10, 20) mit einem Gewinde, das das Gewinde des Kolbenstabes (9) derart eingreift, daß die Gesamtlänge des Kolbenstabes (9) mit dem Dosiseinstellelement (10, 20) durch Drehen des Dosiseinstellelements (10, 20) relativ zu dem Kolbenstab (9) eingestellt werden kann,
ein Verriegelungselement (16), das in das offene vordere Ende des Gehäuses (17) eingeschraubt werden kann, wobei Raum für eine an diesem Verriegelungselement (16) montierte Nadel (19) verbleibt,
dadurch gekennzeichnet, daß der Kolbenstab (9) mit dem Dosiseinstellelement (10, 20) axial verschiebbar in dem hinteren Ende des Gehäuses (17, 8) derart montiert ist, daß das hintere Ende des Kolbenstabes (9) mit dem Dosiseinstellelement (10, 20) durch das hintere Ende des Gehäuses herausgedrückt wird, wenn die Patrone (1) durch das offene vordere Ende ihres Gehäuses (17) eingesetzt wird, wobei ihr erster Kolben (23) gegen das vordere Ende des Kolbenstabes (9) stößt, um diesen Stab nach hinten zu drücken, wobei die minimale Länge des Kolbenstabes (9) mit dem Dosiseinstellelement (10, 20) derart ist, daß ein Mischen in der vollständig eingesetzten Patrone (1), die in dem Gehäuse (17) durch Schrauben des Verriegelungselements (16) in das offene Ende dieses Gehäuses (17) festgemacht ist, gerade durchgeführt wird, wenn der Kolbenstab (9) mit dem Dosiseinstellelement (10, 20) in das Gehäuse hineingedrückt wird, bis das Dosiseinstellelement (10, 20) an einen Anschlag an dem Gehäuse (17) stößt.

2. Eine Spritze nach Anspruch 1, dadurch gekennzeichnet, daß eine Rückzugarretierung (4, 25) vorgesehen und betätigt wird, wenn eine Patrone in das Gehäuse (17) eingesetzt wird.

3. Eine Vorrichtung nach Anspruch 1 oder 2, dadurch gekennzeichnet, daß durch Injektion nach dem Mischen die Vorwärtsbewegung des Kolbenstabes (9) durch das Dosiseinstellelement (10, 20), das von dem hinteren Teil des Kolbenstabes (9) losgeschraubt ist, voreingestellt wird, so daß die Entfernung zwischen dem Dosiseinstellelement (10, 20) und den Anschlag an dem Gehäuse (17) durch Drehen des Dosiseinstellelements (10, 20) relativ zu dem Kolbenstab (9) eingestellt wird.

4. Eine Spritze nach Anspruch 3, dadurch gekennzeichnet, daß eine Skala (7) von einer Feder (12) in Richtung einer Nullposition in Bezug zu dem Gehäuse (17) vorgespannt und mit dem Dosiseinstellement (10, 20) gekoppelt ist, um der Drehung dieses Elements zu folgen, wenn es gedreht wird, um es von seinem Anstoßen an dem Anschlag wegzuschrauben, wobei die Kopplung gelöst wird, wenn das Dosiseinstellelement zum Anstoßen mit dem Anschlag an dem Gehäuse (17) hineingedrückt wird.

5. Eine Spritze nach Anspruch 3, dadurch gekennzeichnet, daß der Kolbenstab (9) durch eine Kupplung (28, 29) während des Drehens des Dosiseinstellelements (10, 20) eingekuppelt ist.

6. Vorrichtung nach Anspruch 2, 3 und 5, dadurch gekennzeichnet, daß die Rückzugarretierung (4, 25) und die Kupplung (28, 29) als Halter (28) mit Armen (4) vorgesehen sind, wobei Zähne nach hinten zeigende Zähne (25) an dem Kolbenstab (9) eingreifen, wodurch der Kolbenstab an einer Seite daran gehindert wird, nach hinten geleitet zu werden, wenn die Arme (4) gegen den Kolbenstab (9) gedrückt werden, und an der anderen Seite daran gehindert wird, gedreht zu werden, wenn die Halter (28) längliche Schienen (29) in dem Kolbenstab (9) eingreifen.

## Revendications

1. Seringue destinée à mélanger un liquide et un médicament dans une cartouche à deux chambres (1) comprenant un cylindre avec son extrémité avant fermée par une membrane, son extrémité arrière fermée par un piston (23) et son espace intérieur divisé en deux compartiments par un second piston (24) et destinée ensuite à une injection dosée du mélange, cet appareil comprenant
un logement tubulaire (17, 8) avec une extrémité avant ouverte et une extrémité arrière,
une tige de piston (9) avec une extrémité avant prévue pour s'engager avec le premier piston (23) de la cartouche (1), et une extrémité arrière avec un filetage,
un élément de dosage (10, 20) avec un filetage s'engageant dans le filetage de la tige de piston (9) de sorte que la longueur totale de la tige de piston (9) avec l'élément de dosage (10, 20) peut être ajustée par mise en rotation de l'élément de dosage (10, 20) par rapport à la tige de piston (9),
Un élément de blocage (16) pouvant être vissé dans l'extrémité avant ouverte du logement (17) laissant de l'espace pour une aiguille (19) montée sur cet élément de blocage (16),
caractérisée en ce que la tige de piston (9) avec l'élément de dosage (10, 20) est montée de façon déplaçable axialement dans l'extrémité arrière du logement (17, 8) de sorte que l'extrémité arrière de la tige de piston (9) avec l'élément de dosage (10, 20) est poussée à l'extérieur par l'extrémité arrière du logement lorsque la cartouche (1) est introduite par l'extrémité avant ouverte de ce logement (17) avec son premier piston (23) venant en butée sur l'extrémité avant de la tige de piston (9) pour comprimer ce piston vers l'arrière, la longueur minimum de la tige de piston (9) avec l'élément de dosage (10, 20) étant telle que le mélange ne s'effectue que dans la cartouche (1) parfaitement introduite et serrée dans le logement (17) par vissage de l'élément de blocage (16) dans l'extrémité ouverte de ce logement (17) lorsque la tige de piston (9) avec l'élément de dosage (10, 20) est comprimée dans sa position initiale dans le logement jusqu'à ce que l'élément de dosage (10, 20) vienne en arrêt sur une butée du logement (17).

2. Seringue selon la revendication 1, caractérisée en ce qu'il est prévu un positionneur de retrait (4, 25) actionné lorsqu'une cartouche est introduite dans le logement (17).

3. Appareil selon la revendication 1 ou 2, caractérisé en ce que par injection après le mélange, le mouvement avant de la tige de piston (9), est préétabli par l'élément de dosage (10, 20) qui est dévissé de la partie arrière de la tige de piston (9) de sorte que la distance entre l'élément de dosage (10, 20) et la butée sur le logement (17) est fixée par mise en rotation de l'élément de dosage (10, 20) par rapport à la tige de piston (9).

4. Seringue selon la revendication 3, caractérisée en ce qu'une échelle graduée (7) est précontrainte par un ressort (12) sur une position zéro par rapport au logement (17) et elle est raccordée à l'élément de dosage (10, 20) pour suivre la rotation de cet élément lorsqu'il est mis en rotation pour le dévisser de son appui avec la butée, l'accouplement étant libéré lorsque l'élément de dosage est comprimé sur sa position initiale en appui avec la butée sur le logement (17).

5. Seringue selon la revendication 3, caractérisée en ce que pendant la rotation de l'élément de dosage (10, 20), la tige de piston (9) est en prise avec un accouplement (28, 29).

6. Appareil selon les revendications 2, 3 et 5, caractérisé en ce que le positionneur de retrait (4, 25) et l'accouplement (28, 29) sont prévus sous forme de supports (28) avec des bras (4) et des dents s'engageant sur l'arrière des dents en regard (25) sur la tige de piston (9) de sorte que la tige de piston sur un côté est empêchée de revenir en arrière lorsque les bras (4) sont comprimés contre la tige de piston (9) et de l'autre côté elle est empêchée de tourner lorsque les supports (28) s'engagent dans les sillons longitudinaux (29) de la tige de piston (9).
